# EUROPEAN PATENT SPECIFICATION

(11) **EP 3 823 568 B1**
(45) Date of publication and mention of the grant of the patent: **28.10.2026**
(21) Application number: 19838354.9
(22) Date of filing: 15.07.2019
(51) Int. Cl.: A61B 3/16, A61F 9/008, A61F 9/007, A61B 18/20, G06F 17/13

(54) **SURGICAL TREATMENT FOR GLAUCOMA**
CHIRURGISCHE BEHANDLUNG FÜR GLAUKOM
TRAITEMENT CHIRURGICAL DU GLAUCOME

(30) Priority: 16.07.2018 US 201862698551 P
(43) Date of publication of application: 26.05.2021
(73) Proprietor: The Regents of the University of California, Oakland, CA 94607-5200 (US)
(72) Inventor: JUHASZ, Tibor, Oakland, California 94607-5200 (US); JESTER, James, Oakland, California 94607-5200 (US); MIKULA, Eric, Oakland, California 94607-5200 (US)
(74) Representative: Hertin und Partner Rechts- und Patentanwälte PartG mbB
(86) International application number: PCT/US2019/041834
(87) International publication number: WO 2020/018436

(56) References cited:
- WO-A1-2018/049246
- US-A1- 2012 283 557
- US-A1- 2013 103 011
- US-A1- 2015 366 706
- DONGYUL CHAI ET AL: "The Effects of Femtosecond Laser Created Drainage Channels on the Aqueous Humor Outflow Dynamics of the Eye", 1 January 2008 (2008-01-01), XP055676335, Retrieved from the Internet <URL:https://pdfs.semanticscholar.org/16c4/f88ce928af24aef82615cfc9191778ce8dcc.pdf>
- CHAI DONGYUL ET AL: "3D finite element model of aqueous outflow to predict the effect of femtosecond laser created partial thickness drainage channels", LASERS IN SURGERY AND MEDICINE., vol. 40, no. 3, 1 March 2008 (2008-03-01), US, pages 188 - 195, XP093125222, ISSN: 0196-8092, DOI: 10.1002/lsm.20608
- DONGYU, CHAI, THE EFFECTS OF FEMTOSECOND LASER CREATED DRAINAGE CHANNELS ON THE AQUEOUS HUMOR OUTFLOW DYNAMICS OF THE EYE, 2008, XP055676335, Retrieved from the Internet <URL:https://pdfs.semanticscholar.org/16c4/f88ce928af24aef82615cfc9191778ce8dcc.pdf> [retrieved on 20190916]

## Description

### Field

The disclosure relates to a medical device used in ophthalmology and surgical treatment of glaucoma. Surgical treatment of glaucoma generally aims for increasing the aqueous humor (AH) outflow, therefore decreasing the intraocular eye pressure.

### Statement Regarding Federally Sponsored Research

This invention was made with government support under Grant Nos. RO1 EY014456-01 and RO1 EY030304-01, awarded by the National Institutes of Health (NIH). The government has certain rights in the invention.

### Background

An array of glaucoma surgeries are available, including Conventional Filtration Surgery, Minimally Invasive Glaucoma Surgery (MIGS), Argon Laser Trabeculoplasty, (ALT), Selective Laser Trabeculoplasty (SLT) and Excimer Laser Trabeculostomy (ELT).

*Conventional Filtration Surgery:* Glaucoma filtration surgery has been commonly used for over 40 years. Though with the arrival of intraocular pressure (IOP) regulating drugs and less invasive laser procedures, filtration surgery is only used as a last resort when the risks to vision outweigh the risks of surgery (Shaarawy T, et al. 2015 Surv Ophthalmol 60:327-345). The most common filtration surgery is penetrating trabeculectomy, whereby a partial thickness sclera flap is cut after which an opening into the anterior chamber (AC) is created. The flap is sutured back into place thus forming a subconjunctival bleb into which AH drains and filters out of the eye. This and similar procedures lower IOP, but are associated with hypotony (24%), flat AC (24%), hyphema (25%), and bleb leak (18%) (Shaarawy T, et al. 2015 Surv Ophthalmol 60:327-345). The high rate of complications is a significant limitation to conventional surgery. Bleb fibrosis and filtration failure rate is roughly 50% at 5 years (Iverson SM, et al. 2015 Jpn Ophthalmol 59:179-186). Another popular conventional surgery is the implantation of a shunt. Much like trabeculectomy, the sclera is partially dissected and a shunt is inserted linking the AC to the subconjunctival space, where a bleb is formed. The tube versus trabeculectomy study (TVT) revealed a 29.8% and 46.9% failure rate at 5 years for tube and trabeculectomy procedures, respectively (Gedde, SJ, et al. 2012 Am J Ophthalmol 153:789-803 e782). Additionally, the outflow rate increase is not adequately controlled with the filtration surgery.

*Minimally Invasive Glaucoma Surgery (MIGS):* Recently, MIGS have gained favor as they can be performed ab-interno or ab-externo without disrupting the sclera and conjunctiva. This qualification stands in stark contrast to traditional filtration procedures, such as mechanical trabeculectomy and subconjunctival drainage shunts in which the full thickness of the sclera and outflow structures are manipulated. Many MIGS procedures involve the implantation of small stents traversing from the AC into the Schlemm's canal (SC). These devices are typically very small, on the order of 1 mm, and provide a direct route from the anterior chamber to the collector channels. One such device is the iStent (Glaukos Corporation, San Clemente, CA), which has been shown to reduce IOP by 17% at 15 months post-implantation (Richter GM, et al. 2016 Clin Ophthalmol 10: 189-206). The Trabectome (Neomedlx, Tustin CA, USA) also qualifies as a MIGS procecure. The Trabectome is a trabeculectomy procedure in which a strip of trabecular meshwork (TM) and the inner wall of the SC (60-120°) are removed via an electrocautery hand piece, thus providing AH direct access to collector channels (Richter GM, et al. 2016 Clin Ophthalmol 10: 189-206). Though designated as minimally invasive, these examples still require a clear, corneal incision. Cyclophotocoagulation (CPO) is another MIGS procedure during which laser light is delivered either trans-sclerally or gonioscopically to destroy portions (270-360°) of the clliary body to reduce AH secretion (Seibold LK, et al. 2015 J Ophthalmal 22:18-24).

*Argon Laser Trabeculoplasty, (ALT):* Though its use has decreased in the last decade, a common surgical intervention is often a laser-based procedure called argon laser trabeculoplasty, (ALT), in which the TM is ablated with the goal of increasing AH outflow facility. ALT was historically considered in patients with primary open angle glaucoma (POAG) requiring lower IOP with maximum tolerated drug therapy. Studies have indicated that initial ALT for the treatment of POAG can be as efficient as medications, with approximately 80% of the treated eyes showing a drop in IOP of 7 to 13 mm Hg (Odberg, T. et al. 1999 Acta Ophthalmologica Scandinavica 77: 176-181; Group GLTR. 1995 "The Glaucoma Laser Trial (GLT) and glaucoma laser trial follow-up study: 7. Results" Am J Ophthalmol 120:718-731). As with drug treatments, the primary problem with ALT is that effectiveness is relatively short lived (Weber, PA, et al. 1989 Ophthalmic Surg 20:702-706; Feldman RM, et al. 1991 Ophthalmology 98:1061-1065) and questionable after failed medical drug therapy (AGIS I. 2002 American Journal of Ophthalmololgy 134:481-498). The mechanism of action in ALT is thought to be laser mediated burns in the TM which in turn causes collagen and tissue contraction. This is thought to increase AH outflow into Schlemm's canal (Rodrigues, MM, et al. 1982 Ophthalmology 89:198-210; Kagan DB,et al. 2014 Clin experiment Ophthalmol 42:675-681).

*Selective Laser Trabeculoplasty (SLT):* SLT has gained popularity in the past decade as an alternative to ALT. This procedure utilizes a Q-switched ND:YAG laser to deliver nanosecond pulses of 532 nm light to the trabecular meshwork (Latina MA, 2005 Ophthalmol Clin North Am 18:409-419). The light "selectively" targets the pigmented cells of the trabecular meshwork causing physical and biochemical alteration to the outflow pathway (Kagan DB, et al. 2014 Clin experiment Ophthalmol 42:675-681). There is some evidence suggesting progenitor like cells in the anterior non-filtering portion of the TM (near Schwalbe's line) are recruited in response to trabecular meshwork injury. These cells move in and replace the TM cells damaged during SLT. These new TM cells in turn release cytokines responsible for signaling the Schlemm's canal endothelial cells to increase hydraulic conductivity. It is thought that the effect of SLT is largely biological as the laser does not create a physical channel through which the AH can filter, but rather stimulates an increase in permeability of the inner wall of Schlemm's canal (Kagan DB, et al. 2014 Clin experiment Ophthalmol 42:675-681). However, SLT is associated with increased anterior chamber reactions and a limited time window for effectiveness (Latina MA, et al. 1995 Experimental Eye Research 60:359-371; Damji KF, et al. 1999 The British Journal of Ophthalmology 83: 718-722). Three studies reviewing the efficacy of SLT have found an average time to failure of 18 months (Ayala M, et al. 2011 Clin Ophthalmol 5: 573-576; Ayala M, et al. 2011 Open Ophthalmol J 5:32-34; and Ayala M. et al. 2014 J Glaucoma 23:616-619) while another revealed a 25% success rate at 5 years (Lai J.S. et al. 2004 Clin Experiment Ophthalmol 32:368-372). By comparison, argon laser trabeculoplasty was found to have a success rate of 59% at three years and 50% at 5 years (Grinich, N.P. et al. 1987 Ophthalmology 94:858-861; Bovell AM, et al. 2011 Can J Ophthalmol 46:408-413).

*Excimer Laser Trabeculostomy (ELT):* A 308 nm excimer laser is fiber coupled and invasively inserted into the anterior chamber through a corneal incision. Then, the optical fiber is pushed against the trabecular meshwork and the excimer laser beam is used to photo-ablate channels from the AC into the SC through the TM. ELT has resulted in long lasting IOP reduction, however the procedure is invasive and lacks control over the amount of tissue ablation and thus IOP reduction (Berlin MS, et al. 2009 Glaucoma Today*;* Berlin MS, et al. 1987 Am J Ophthalmol 103:713-714).

In summary, there are numerous surgical techniques available to increase the AH outflow, and therefore decrease the IOP. Although some of these surgical techniques provide temporary IOP reduction, all of them lack precise control over the magnitude of the reduction. Relevant published applications and issued patents are listed below in Tables 1 and 2. Also relevant is PCT/US2017/050799 "GLAUCOMA TREATMENT METHODS AND APPARATUS".

**TABLE 1**

| **U.S. Application Publication Number** | **Title** |
|---|---|
| 2017-0127938 | SYSTEMS AND METHODS FOR OBTAINING LOW-ANGLE CIRCUMFERENTIAL OPTICAL ACCESS TO THE EYE |
| 2012-0283557 | METHOD AND APPARATUS FOR THE TREATMENT OF GLAUCOMA USING VISIBLE AND INFRARED ULTRASHORT LASER PULSES |
| 2016-0095751 | METHODS AND APPARATUSES FOR THE TREATMENT OF GLAUCOMA USING VISIBLE AND INFRARED ULTRASHORT LASER PULSES |
| 2015-0313759 | SYSTEMS AND METHODS TO DELIVER PHOTODISRUPTIVE LASER PULSES INTO TISSUE LAYERS OF THE ANTERIOR ANGLE OF THE EYE |
| 2015-0305940 | SYSTEMS AND METHODS TO DELIVER PHOTODISRUPTIVE LASER PULSES INTO TISSUE LAYERS OF THE ANTERIOR ANGLE OF THE EYE |
| 2015-0305939 | SYSTEMS AND METHODS TO DELIVER PHOTODISRUPTIVE LASER PULSES INTO TISSUE LAYERS OF THE ANTERIOR ANGLE OF THE EYE |
| 2015-0157505 | DEVICES AND METHODS FOR GENERATION OF SUBSURFACE MICRO DISRUPTIONS FOR OPTHALMIC SURGERY AND APPLICATIONS |
| 2015-0077528 | SURGICAL NAVIGATION SYSTEM AND METHOD |
| 2002-0013572 | DELIVERY SYSTEM AND METHOD OF USE FOR THE EYE |
| 2014-0216468 | BI-RADIAL PATIENT INTERFACE |
| 2012-0303007 | SYSTEM AND METHOD FOR USING MULTIPLE DETECTOR |

**TABLE 2**

| **U.S. Patent Number** | **Title** |
|---|---|
| 9,033,963 | SYSTEMS AND METHODS TO DELIVER PHOTODISRUPTIVE LASER PULSES INTO TISSUE LAYERS OF THE ANTERIOR ANGLE OF THE EYE |
| 8,230,866 | SYSTEMS AND METHODS FOR TREATING GLAUCOMA AND SYSTEMS AND METHODS FOR IMAGING A PORTION OF THE EYE |
| 9,642,746 | GLAUCOMA SURGERY METHODS AND SYSTEMS |
| 8,679,089 | GLAUCOMA SURGERY METHODS AND SYSTEMS |
| 9,629,750 | SURGICAL LASER UNIT WITH VARIABLE MODES OF OPERATION |
| 9,517,006 | SYSTEMS AND METHODS FOR OBTAINING LOW-ANGLE CIRCUMFERENTIAL OPTICAL ACCESS TO THE EYE |
| 7,282,046 | GLAUCOMA TREATMENT METHOD |
| 8,568,393 | COMPUTER GUIDED LASER TRABECULOPLASTY |
| 9,681,985 | SYSTEM AND METHOD FOR MINIMALLY TRAUMATIC OPHTHALMIC PHOTOMEDICINE |
| 9,498,295 | OPTIMIZATION OF LASER THERAPY |
| 9,554,702 | MICROSCOPE-LESS WIDE FIELD-OF-VIEW SURGICAL OCT VISUALIZATION SYSTEM |
| 8,920,407 | OPTICAL SYSTEM FOR OPHTHALMIC SURGICAL LASER |
| 9,320,650 | METHOD AND APPARATUS FOR PRECISION WORKING OF AN EYE |
| 8,171,937 | METHOD AND APPARATUS FOR PRECISION WORKING OF MATERIAL |
| 7,351,241 | METHOD AND APPARATUS FOR PRECISION WORKING OF MATERIAL |
| 7,131,968 | APPARATUS AND METHOD OF OPHTHALMOLOGIC SURGICAL PROCEDURES USING A FEMTOSECOND FIBER LASER |
| 9,724,238 | OPHTHALMIC INTERFACE APPARATUS, METHOD OF INTERFACING A SURGICAL LASER WITH AN EYE, ANDSUPPORT RING FOR USE WITH A SUCTION RING |
| 8,845,624 | ADAPTIVE PATIENT INTERFACE |
| 8,523,926 | DEVICES AND METHODS FOR GENERATION OF SUBSURFACE MICRODISRUPTIONS FOR BIOMEDICAL APPLICATION |
| 6,482,199 | METHOD AND APPARATUS FOR HIGH PRECISION VARIABLE RATE MATERIAL REMOVAL AND MODIFICATION |

### SUMMARY

We disclose a device and a method for surgical treatment of glaucoma. Methods referred to and described here relating to surgical or therapeutic methods of treatment are not part of the invention.

The invention is defined by the appended claims.

The invention therefore relates to a glaucoma treatment apparatus comprising:
an imaging device configured to capture pre-operative images of the anterior segment of the eye,
a femtosecond (FS) laser configured to actuate at a wavelength of about 1030 nm and apply laser pulses to the trabecular meshwork (TM) and the juxtacanicular tissue (JCT) of the eye, and
a hardware processor configured to:
   receive a pre-operative intraocular pressure (IOP) level and a target IOP reduction; and
   determine optimal location(s) and cross sectional area(s) of one or more aqueous humor (AH) drainage channels through the trabecular meshwork (TM) and the juxtacanalicular tissue (JCT) of the eye based on at least:
      an anatomy of the anterior segment of the eye obtained from pre-operative images of the anterior segment of the eye, said anatomy including the TM, the JCT, the Schlemm's Canal (SC) and collector channels (CCS),
      a pre-operative intraocular pressure (IOP) level, and
      a target IOP reduction,
      wherein the hardware processor is configured to determine the optimal location(s) and cross-sectional area(s) of the one or more AH drainage channel(s) to be created by the FS laser in order to achieve the target IOP reduction by using a three-dimensional finite element model (FEM) computer model of AH outflow dynamics that uses the anatomy of the anterior segment of the treated eye, including the TM, SC and CCS obtained from pre-operative images, the measured IOP, and target IOP reduction as inputs,
   wherein the FEM model assumes the following:
      (i) all AH outflow occurs through the trabecular pathway;
      (ii) the trabecular pathway in the eye is porous, so that outflow through the pathway follows the Brinkman equation and its resistance to outflow is characterized with permeability in the equation: $- v \left[η\left(v{u}_{_}+{\left(v{u}_{_}\right)}^{r}\right)\right] + vP + \frac{η}{K} {u}_{_} = 0 , v {u}_{_} = 0 ,$ where v is a differential operator, u is a velocity vector (m/s), η is the dynamic viscosity in (Pa*second), P is pressure, (vu)r refers to the transposed velocity gradient vector and κ is permeability in m²;
      (iii) the resistance of pathways through the TM, juxtacanalicular tissue (JCT) and inner wall endothelium of the SC are characterized by different permeability values;
      (iv) the structures of the eye such as the cornea are rigid materials, and for the precise estimation of the AH outflow parameters deformation of the cornea and movement of the structural components such as lens and iris inside the eye are included in the FEM of the AH outflow; and
      (v) impact of temperature variation on the AH outflow is not considered;
wherein the FEM is configured to determine the three-dimensional aqueous humor outflow dynamics of the pre-operative eye and the average permeability of the trabecular meshwork,
wherein the hardware processor is configured to use an inverse of the FEM to calculate the locations and necessary cross-sectional area(s) of the channel(s) to achieve the target IOP reduction; and
wherein the hardware processor is configured to actuate the laser in order to create the one or more drainage channel(s) through the TM and the JCT with the cross-sectional area(s) and location(s) determined in order to achieve the target intraocular pressure (IOP) reduction.

Also disclosed herein is a glaucoma treatment apparatus comprising:
an imaging device configured to image an anterior segment of the eye,
a laser, and
a hardware processor configured to:
   determine a location and a cross sectional area of a treatment based on an anatomy of the anterior segment of the eye obtained from pre-operative images of the anterior segment of the eye, a pre-operative intraocular pressure (IOP) level, and a target IOP reduction, said anatomy including the trabecular meshwork (TM), the Schlemm's Canal (SC) and collector channels (CCS), and
   actuate the laser to create an outflow channel with the cross-sectional area and location in order to achieve the target intraocular pressure (IOP) reduction.

In some embodiments, the glaucoma treatment apparatus further includes a suction ring configured to keep the apparatus steady on the eye during a treatment procedure.

In some embodiments, the apparatus is configured to automatically create drainage channels in between the AC and the SC, based on output from the hardware processor.

In some embodiments, the treatment laser has a pulse duration ranging from 50 fs to 50 ns.

In some embodiments, the treatment laser has a wavelength ranging from 400 nm to 2000nm.

In some embodiments, the treatment laser has a repetition rate ranging up to 500 KHz.

In some embodiments, the imaging device is selected from the group consisting of a light microscope, an ultrasound device, a computed tomography (CT) device, a magnetic resonance imager (MRI), a system that utilizes molecular imaging, a system that utilizes nuclear medicine, a positron emission tomography (PET) system, a radiography system, and a fluoroscopy system.

For illustrative purpose a method of reducing intraocular pressure in an eye is described, including:
acquiring a pre-operative image of the anterior segment of the eye,
using an algorithm based on the customized anatomy of the anterior segment including the TM, SC and CCS obtained from a pre-operative images of the anterior segment of the eye, a pre-operative IOP level and a target IOP reduction as an inputs, to determine a location and a cross-sectional area of treatment or various locations and a cross-sectional area of multiple treatments in the trabecular meshwork of the eye that will allow precise personalized control over intraocular pressure reduction,
targeting the trabecular meshwork with laser pulses of short durations to create drainage channels in between the anterior chamber (AC) and the Schlemm's canal (SC), and
draining of aqueous humor from the anterior chamber to the SC to reduce intraocular pressure in the eye, wherein the channels mediate precise and customized control over the magnitude and/or rate of drainage of the aqueous humor into the SC.

In some examples of the method, the laser pulses of short durations used for the treatment range from 50 fs to 50 ns.

In some examples of the method, wavelength(s) of the laser pulses of short durations used for the treatment range from 400 nm to 2000 nm.

In some examples of the method, a repetition rate of the laser pulses of short durations used for the treatment ranges up to 500 KHz.

In some examples of the method, in the algorithm, the anterior chamber is modeled as a hemisphere.

In some examples of the method, the hemisphere is modeled with a diameter of 12 mm along the edge of the iris.

In some examples of the method, in the algorithm, the height of the trabecular meshwork (TM) is modeled to range from 0.38 to 0.86 mm.

In some examples of the method, in the algorithm, the height of the trabecular meshwork (TM) is modeled to have an average height of 0.6 mm.

In some examples of the method, in the algorithm, the final destination of aqueous humor (AH) flow is modeled to have a constant pressure boundary condition of 9.6 mmHg, corresponding to the back-pressure in the episcleral venous system.

In some examples of the method, the algorithm is based on the Navier-Stokes equation and continuity equations, where the AH is described as incompressible fluid, modified to describe steady state flow and to describe AH dynamics in the model: $- v \left[1\left(v{u}_{_}+{\left(v{u}_{_}\right)}^{r}\right)\right] + {puvu}_{¯} + {vP}_{¯} = 0 , v {u}_{_} = 0 ,$ and wherein flow through the trabecular meshwork is governed by the steady state Brinkman equation: $- v \left[1\left(v{u}_{_}+{\left(v{u}_{_}\right)}^{r}\right)\right] + {vP}_{¯} + \frac{1}{K} {u}_{_} = 0 , v {u}_{_} = 0$ where µ is velocity (m/s), ρ is density (kg/m3), η is dynamic viscosity (Pa* second), F is body force (N), and k is permeability (m2).

The exemplary use of the glaucoma treatment apparatus is described herein to target the trabecular meshwork with laser pulses of short durations to create drainage channels in between the anterior chamber (AC) and the Schlemm's canal (SC), wherein dimensions of the drainage channels and positioning of the drainage channels are calculated using an algorithm based on a customized anatomy of the anterior segment of the eye including the TM, SC and CCS obtained from the pre-operative images of the anterior segment of the eye, a pre-operative IOP level and a target IOP reduction as inputs to determine a location and a cross-sectional area of treatment or various locations and a cross-sectional area of multiple treatments in the trabecular meshwork of the eye that will allow precise personalized control over intraocular pressure reduction.

### BRIEF DESCRIPTION OF THE DRAWINGS

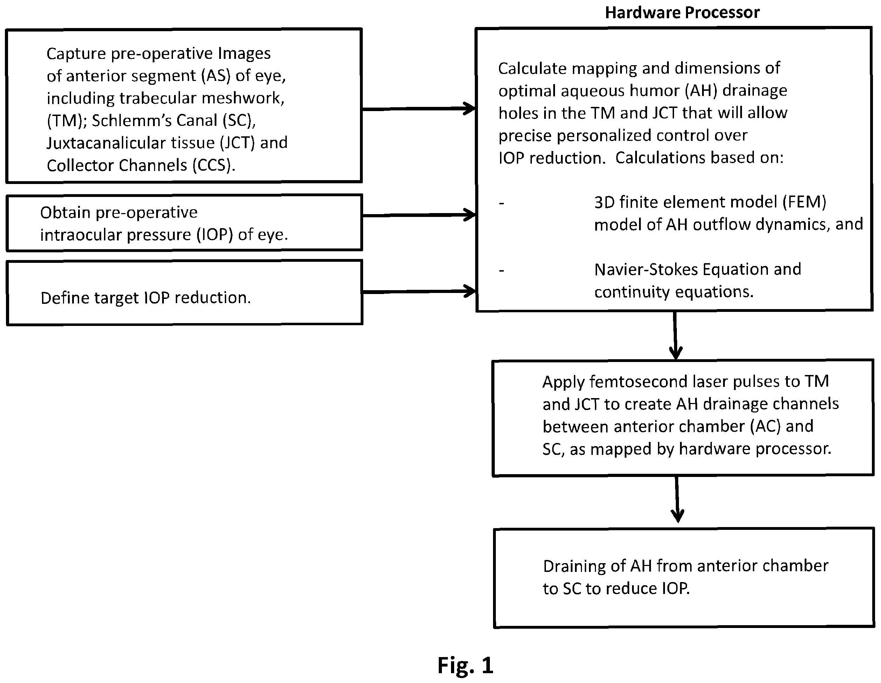

Figure 1. Flow scheme for mapping locations and dimensions of optimal aqueous humor (AH) drainage holes in the TM and JCT to allow precise personalized control over IOP reduction.
Figure 2. Treated eyes demonstrate a reduction from pre-operative intraocular pressure (IOP).
Figure 3. (A) shows the general experimental setup used, demonstrating ab-interno creation of drainage channels with a goniolens. (B) A second harmonic generation (SHG) image of the drainage channel. (SC-Schlemm's canal, TM-trabecular meshwork).
Figure 4. Schematic of the eye.
Figure 5. (a) Schematic of outflow pathway used in 2D model. (b) Modified 2D model.
Figure 6. Geometry of 3D model.
Figure 7. Profile of the AH flow in the 2D model in the simplified geometry shown in Figure 5(b). Color code represents velocity of AH.
Figure 8. The AH flow through the channel as shown by the red lines.

### DETAILED DESCRIPTION

Despite the wide array of available glaucoma surgeries, the limitations of current procedures drive the need for a better alternative. These limitations include: invasiveness of the procedures, relatively quick healing and therefore short efficacy of the procedures, and complete lack of precise control of the IOP reduction. Therefore, future glaucoma treatment technology would benefit from reduced collateral damage and wound healing responses, as well as an increase in spatial precision and precise control over the target IOP reduction. Overall, the next generation glaucoma surgery needs to have the following attributes: 1) be non-invasive to preserve the conjunctival sclera, and cornea, 2) minimize collateral damage to tissue surrounding the treatment site; 3) have high precision (µm scale) to finely control the geometry of the treated tissue and thus the AH outflow increase; 4): be customized and be able to utilize an algorithm based on the anatomy of the anterior segment of the eye including the TM, SC and CCS, a pre-operative IOP as inputs that relates the treatment size with a target IOP reduction providing a better control over the surgical outcome than current technologies. Such treatments may be performed quickly in an outpatient setting.

Current Femtosecond (FS) Laser Technology and its Anterior Segment Applications: High power femtosecond lasers were developed in the early nineties (Salin F, et al. 1991 Opt Lett 16: 1964-1966; Squier JA, et al. 1993 Opt Lett 18:625), though early systems were large, expensive and unreliable for practical applications, advances in ultrafast laser design and solid-state laser pumping sources made femtosecond lasers viable clinical instruments in the late nineties (Sletten KR, et al. 1999 Ophthalmic Surg Lasers 30:742-749). The first clinical femtosecond laser was developed exclusively for corneal resections in collaboration between the University of Michigan and IntraLase Corporation under NIH SBIR R44 EY1234-02 (Ratkay-Traub, I. et al. 2001 Ophthalmol Clin North Am 14:347-355; Ratkay-Traub, I. et al. 2003 J Refract Surg 19:94-103; and Juhasz T, et al. 1999 Selected Topics in Quantum Electronics, IEEE Journal of 5:902-910). This system was cleared by the FDA in 2001 and has become the standard of care for several corneal procedures (Kim P, et al. 2011 Curr Opin Ophthalmol 22:238-244). OCT guided femtosecond laser technology was developed for performing surgical incisions in cataract surgery (Sandor GL, et al. 2014 J Refract Surg 30:660-664; Nagy ZZ, et al. 2013 J Refract Surg 29:8; Nagy ZZ, et al. 2013 J Refract Surg 29:110-112; Juhasz E, et al. 2014 J Refract Surg 30:622-626; Juhasz T. 2012 Laser Refractive Cataract Surgery: BMC; pages 75-83; Juhasz T. 2012 "Challenges for Femtosecond Laser Technologies for Cataract Surgery" Femtosecond Laser Refractive Cataract Surgery: Springer; pages 39-48 and Juhasz T. Competing Femtosecond Laser technologies for Cataract Surgery. In: Nagy ZZ (ed) Femtosecond Laser Assisted Cataract Surgery: Facts and Results: Slack; 2014 3-10).

This system was also cleared by the FDA and is now widely used to perform capsulotomy, lens fragmentation and corneal incisions during cataract surgery Sandor GL, et al. 2014 J Refract Surg 30:660-664; Nagy ZZ, et al. 2013 J Refract Surg 29:8; Nagy ZZ, et al. 2013 J Refract Surg 29:110-112, Nagy ZZ, et al. 2014 J Cataract Refract Surg 40:20-28; Nagy ZZ, et al. 2015 Eye Vis (Land) 2:11; Nagy ZZ, et al. 2011 J Refract Surg 27:564-569; and Nagy ZZ, et al. 2015; Orv Hetil 156:221-225).

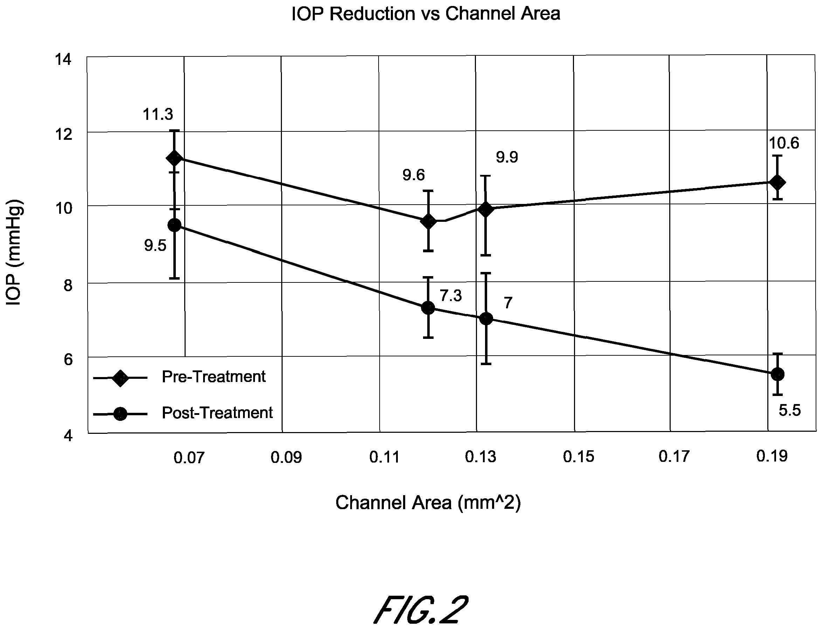

1.7 µm femtosecond laser pulses have been delivered through the translucent conjunctiva and sclera and have been shown to be capable of performing high precision, photodisruptive surgery without damage to superficial or adjacent tissues (Chai, D. et al. 2010 Lasers Surg Med 42:647-651; Sacks ZS, et al. 2003 Ophthalmic Surg Lasers Imaging 34:104-113*;* and Sacks ZS, et al. 2002 J Biomed Opt 7:442-450). Intra-scleral channels created with this method in *ex vivo* human and rabbit eyes have also been shown to increase outflow facility, suggesting the potential to reduce IOP (Chai D., et al. 2008 Lasers Surg Med 40:188-195). In a recent study, we demonstrated that subsurface channels can also be created effectively *in vivo* using a rabbit model. Treated eyes also demonstrated a reduction from pre-operative IOP, suggesting potential clinical utility (Chai, D. et al. 2010 Lasers Surg Med 42:647-651) (Fig. 2). The amount of IOP reduction was shown to scale linearly with the cross-sectional area of the laser created channel, suggesting that modifying the laser channel dimensions can be used to fine tune the reduction. Furthermore, this procedure treated less than 10° of the corneo-scleral rim, thus suggesting potential for retreatment in the remaining 350°. For comparison, procedures such as ALT, SLT, and the Trabectome routinely treat 180°-360° of the angle, thus making retreatment problematic. These studies demonstrated that the creation of subsurface scleral channels of well-defined geometry was possible with 1.7 µm femtosecond laser light. Overall, the procedure was like traditional filtration surgery in that the trabecular meshwork and Schlemm's canal were not precisely targeted. Rather, the channel provided AH access to a thinned scleral region through which it could then filter/percolate.

In a separate *in vitro* study performed in 4 mm excised strips of the porcine corneoscleral rims, it was demonstrated that, by directly targeting the exposed trabecular meshwork (TM), the TM and juxtacanalicular tissue (JCT) can be ablated with 800 nm FS laser pulses with moderate, 7 to 14 µJ, pulse energies in extremely short, 0.5 to 2 second, exposure times (Toyran, S. et al. 2005 Experimental Eye Research 81:298-305). The same group also investigated the feasibility of ablating the TM with FS laser pulses through a gonioscopic contact lens in intact porcine and primate globes, as well as in human cadaver eyes. However, imaging studies of the ablation zone indicated that they failed to ablate through the TM, and did not reach the JCT (Nakamura, H., et al. 2009 Invest Ophthalmol Vis Sci 50: 1198-1204; Liu Y, et al. 2008 Ophthalmic Surg Lasers Imaging 39:485-490). Although perfusion studies were not performed in the treated eyes, it is expected that these treatments would fail to lower the IOP, since the JCT, which is known as the major barrier to the outflow, remained intact.

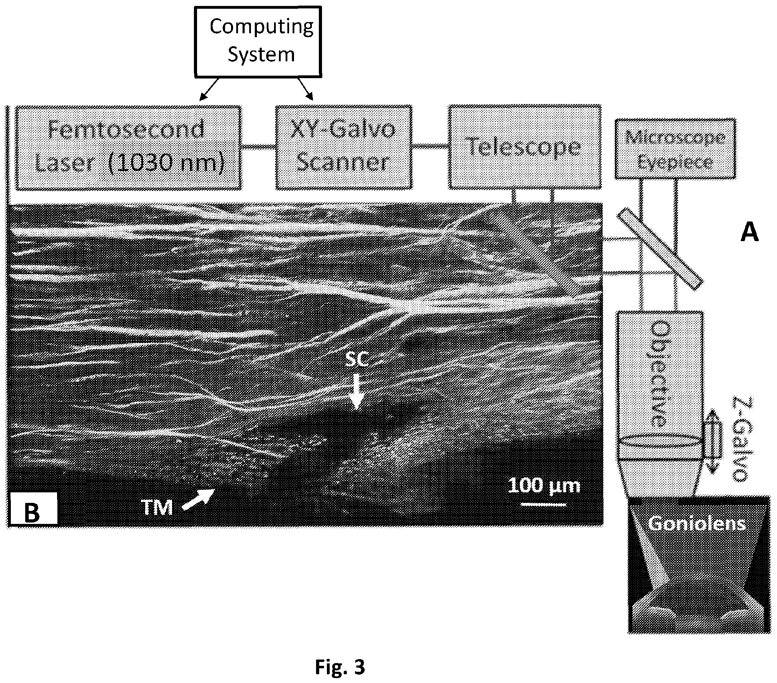

*The potential of FS laser Technology for the treatment of Glaucoma:* The results discussed above suggest the importance of locating and targeting the TM, JCT and SC with extraordinary precision for the creation of AH drainage channels with FS laser pulses. In fact, when the significant technical barriers of precisely locating and targeting these anatomical structures in the intact eye with tightly focused FS laser light are overcome, the TM and JCT can be ablated through with practically no collateral tissue damage in a very short period of time (seconds). In recent tissue studies, we demonstrated that drainage channels reaching the SC can be created with femtosecond laser pulses (See Fig. 3) in human cadaver globes. With reference to Fig. 2, subsurface channels created effectively *in vivo* using a rabbit model resulted in a reduction in post-operative IOP compared to pre-operative IOP.

These studies, as well as the clinical success of FS technology in refractive and cataract surgery indicate that femtosecond technology has the potential to provide a novel treatment for glaucoma with the following advantages:
- The potential of a practically non-invasive procedure with no collateral damage to surrounding tissue, the scleral surface, and conjunctiva, thus potentially reducing post-operative scarring and providing clinically relevant longevity.
- Micron accuracy implementation of customized outflow channel geometry calibrated to achieve a given increase of AH outflow rate.
- Precise control over the IOP reduction by developing an algorithm based on clinical data (to be collected in clinical trials) to determine the cross-sectional area of the treatment.

Since the surgical effect of femtosecond laser pulses is highly precise and the collateral tissue damage is minimal, it is anticipated that the FS laser treatment will provide a precise, long lasting IOP control for a wide range of patients, and therefore, will have significant clinical impact.

We have demonstrated the basic principles of channel creation in the trabecular meshwork in our laboratory. The procedure results in a well-controlled, IOP reduction. Since the cross-section and location of the treatment area is determined by an algorithm based on customized anatomy of the eye obtained from pre-operative images and personalized clinical data, the accuracy of the IOP reduction is better than conventional procedures. Pre-operative images may be acquired with an imaging device, such as a light microscope, ultrasound, computed tomography (CT), magnetic resonance imaging (MRI), molecular imaging, nuclear medicine, positron emission tomography (PET), radiography, and fluoroscopy systems. The femtosecond laser based surgical device described herein performs the procedure completely non-invasively, without any pain and discomfort to the patient, in contrast to performing conventional invasive procedures. The non-invasive nature together with the precise control over the IOP reduction provides a strong competitive advantage to the FS laser based procedure over all the conventional methods.

### Description of algorithm that determines the cross-sectional area of the outflow channel to achieve the target IOP reduction:

To predict the exact size of the drainage channels necessary to create to achieve the target IOP reduction, a novel three dimensional (3D) finite element model (FEM) computer model of the AH outflow dynamics was developed. This novel 3D FEM is advanced to use the anatomy of the anterior segment of the treated eye, including the TM, SC and CCS obtained from pre-operative images, the measured IOP and target IOP reduction as inputs, and calculates the optimal location and cross sectional area of the channels to be created by the FS laser in order to achieve the target IPO. This 3D FEM model is based on the following assumptions:
1. The amount of AH flowing out of the eye through the uveoscleral pathway is not exactly known, but is generally considered to be much less than the outflow through the conventional or trabecular pathway. Therefore, it is assumed that all the AH outflow occurs through the trabecular pathway.
2. The trabecular pathway in the eye is porous, so that outflow through the pathway follows the Brinkman equation and its resistance to outflow can be characterized with permeability in the equation. This assumption is in line with the efforts to quantify the outflow resistance (Barocas VH and Heys JJ. 2001 Invest Ophthalmol Vis Sci 42(4): 3563 Suppl S.), and ultimately to provide regulated decrease in IOP.
3. The resistance of pathways through TM, juxtacanalicular tissue (JCT) and inner wall endothelium of the SC are characterized by different permeability values since it is known that the JCT and inner wall endothelium of the SC are the major barrier to the outflow.
4. The structures of the eye such as the cornea are rigid materials, and for the precise estimation of the AH outflow parameters deformation of the cornea and movement of the structural components such as lens and iris inside the eye are included in the FEM of the AH outflow. Note that the relationship between IOP and movement of eye structures has been investigated earlier (Kumar S, et al. 2006 Ann Biomed Eng 34(3):530-544).
5. The potential impact of temperature on the AH outflow is not considered.

Previous studies demonstrated (Barocas VH and Heys JJ. 2001 *Invest Ophthalmol Vis Sci* 42(4): 3563 Suppl S) that there are two potential heat sources that can have impact on AH flow, the temperature difference between the cornea and the rest of the eye and temperature differences created by the blood flow. These temperature gradients can affect AH flow but are neglected in the current model.

### Mathematical model

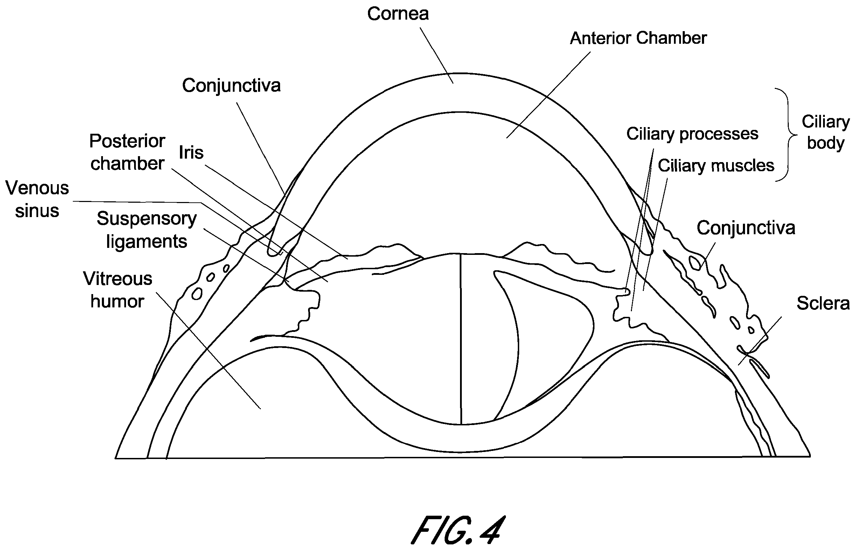

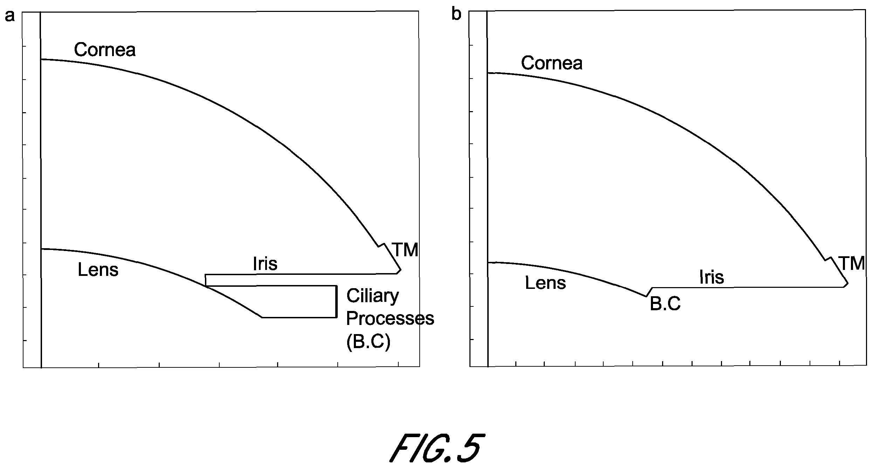

A schematic of the eye is displayed in Figure 4. Our preliminary 2D model was constructed so that it focused on AH flow dynamics in the anterior chamber. It was shown that the gap between the lens and iris is on the order of micrometers and doesn't pose any significant resistance to the flow of AH from the posterior chamber to the anterior chamber of the eye. (Chai D, et al. 2007 Proc SPIE 2007 6435:643500-643501, Sokol J, et al. 1996 Ophthalomology 103(2): 289-293). Thus, this narrow gap needlessly adds to the computational expense of the model without adding any information or validity to the results of the model. Therefore, our model focused on AH dynamics in the anterior chamber. The anterior chamber was modeled as a hemisphere with a diameter of 12 mm along the edge of the iris (Barocas VH and Heys JJ. 2001 Invest Ophthalmol Vis Sci 42(4): 3563 Suppl S.). Alternatively, the diameter of the anterior chamber may range from 11.2 mm to 13.2 mm, for example 11.5 mm, 12 mm, 12.5 mm, or 13 mm. The height of the trabecular meshwork (TM) in human eyes ranges from 0.38 to 0.86 mm (Reitsamer HA and Kiel JW 2002 Invest Opthalmology & Visual Science; 43(12): 3728- 3734). Therefore, we used an average height of 0.6 mm. Alternatively, the height of the TM may be 0.4 mm, 0.45 mm, 0.5 mm, 0.55 mm, 0.6 mm, 0.65 mm, 0.7 mm, 0.75 mm, 0.8 mm or 0.85 mm. In order to simplify the model, the AH inflow was assumed to enter anterior chamber through the velocity inlet marked with a line marked B.C. in Figure 5b, where the gap between the iris and lens would be otherwise. Boundary conditions at the velocity inlet were given so that the inflow rate was the same as those measured in physiological outflow experiments. This eliminates the problems from meshing the narrow iris-lens gap (see Fig. 5, (a) and (b)) for the illustration of the geometry of the 2D model).

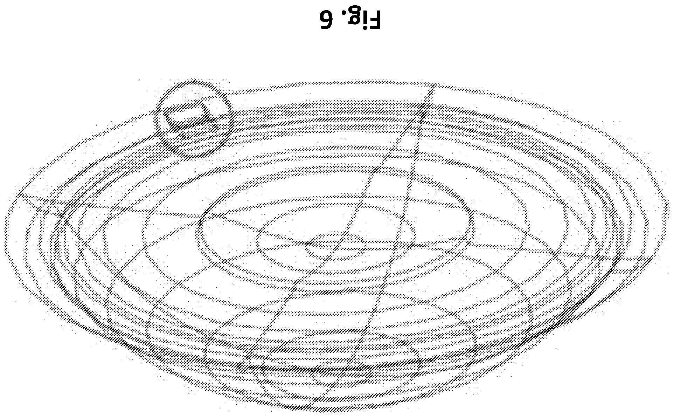

In the living eye, AH flows through the trabecular meshwork, into Schlemm's canal, and then into the collector channels before exiting the eye. Therefore, the final destination of AH flow is modeled to be the constant pressure boundary condition, 9.6 mmHg, corresponding to the back-pressure in the episcleral venous system (Brubaker RF. 1975 Invest Ophthalmol Vis Sci 14(4):286-292). The 3D model is developed by the revolution of the modified axially symmetric 2D model by 360 degrees. The pressure may be between 8-12 mm Hg, including intermediate values of 8 mm Hg, 9 mm Hg, 10 mm Hg, 11 mm Hg and 12 mm Hg. The geometry of 3D model with the femtosecond laser created channel (marked with a circle) is shown in Figure 6. The Navier-Stokes equation and continuity equations, where the AH is described as incompressible fluid were modified to describe steady state flow and used to describe AH dynamics in the model: $- v \left[1\left(v{u}_{_}+{\left(v{u}_{_}\right)}^{r}\right)\right] + {puvu}_{¯} + {vP}_{¯} = 0 , v {u}_{_} = 0$

Flow through the trabecular meshwork is governed by the steady state Brinkman equation: $- v \left[1\left(v{u}_{_}+{\left(v{u}_{_}\right)}^{r}\right)\right] + {vP}_{¯} + \frac{1}{K} {u}_{_} = 0 , v {u}_{_} = 0$ where µ is velocity (m/s), ρ is density (kg/m³), η is dynamic viscosity (Pa* second), F is body force (N), and k is permeability (m²). Each equation was simplified, because we focused on steady state flow in the anterior chamber and in this case the body force is absent. The properties of AH used in model were ρ =1,000 kg/m³, η = 1.002 X 10⁻³ Pa · s. These equations correspond to the stationary flow case.

The equations provided above were solved by COMSOL Multiphysics 3.3 (Comsol, Inc., Burlington, MA) for the finite element mesh and boundary conditions described above. The flow resistance of the trabecular pathway was calculated by fitting the experimental AH outflow data measured in untreated human eyes to the 2D model.

The simulated channel was created through the trabecular meshwork in the 3D model and its flow resistance was assumed to be zero.

The base model calculates the 3D aqueous humor outflow dynamics of the pre-operative eye based on the customized anatomy of the anterior segment including the TM and SC obtained from pre-operative images. Using the pre-operative IOP values as input parameters, the average permeability of the trabecular meshwork is also determined with the help of the model.

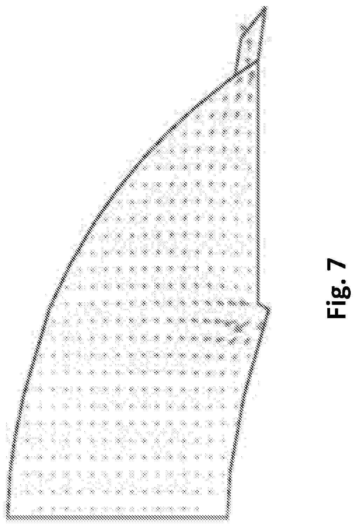

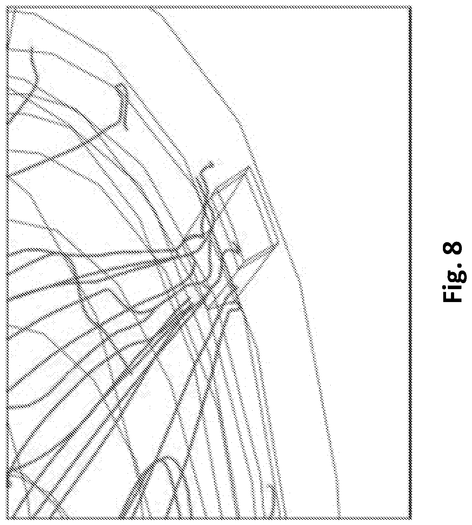

In the final step, the target IOP reduction is used as an input and the inverse model is solved to calculate the necessary cross sectional area (of the femtosecond laser created trabecular drainage channel) to achieve the required IOP reduction for a selected location of the channel. By varying the position of selected location the most efficient position of the treatment location can be found, where a unit cross sectional area of the channel provides the largest IOP reduction. Using data from multiple patients the accuracy of the model can be increased. The results from the 2D and 3D model calculations are shown in Fig. 7 and Fig. 8 respectively.

Based on the modeling and calculations by the computing system, femtosecond laser pulses are applied to the TM and JCT to create AH drainage channels between anterior chamber (AC) and SC, as mapped by hardware processor. Typically, between 1-100 drainage channels are created. In some embodiments, the number of channels is about 1, 2, 4, 10, 20, 40, 60, 80 or 100. Drainage channels with cross sectional shapes can be created, for example circular, rectangular, ovoid, hexagonal and any other geometric shape. The diameter of the drainage channels may vary from 10µm to 600µm, for example 50µm, 100µm, 150µm, 200µm, 250µm, 300µm, 350µm, 400µm, 450µm and 500µm. The height of the channels may vary from 10 µm to 600 µm, for example 50µm, 100µm, 150µm, 200µm, 250µm, 300µm, 350µm, 400µm, 450µm, 500µm and 550µm, and the width may vary from 10 µtm to 10,000 µm, for example 50µm, 100µm, 500µm, 1,000µm, 2,000µm, 3,000µm, 4,000µm, 5,000µm, 6,000µm, 7,000µm, 8,000µm, 9,000µm and 10,000µm. The length of the drainage channels may vary from 100 µm to 400 µm, for example 150µm, 200µm, 250µm, 300µm and 350µm, and it should be sufficient to provide fluid connection in between the AC and the SC.

While the present description sets forth specific details of various embodiments, it will be appreciated that the description is illustrative only and should not be construed in any way as limiting. Furthermore, various applications of such embodiments and modifications thereto, which may occur to those who are skilled in the art, are also encompassed by the general concepts described herein. Each and every feature described herein, and each and every combination of two or more of such features, is included within the scope of the present invention provided that the features included in such a combination are not mutually inconsistent.

Some embodiments have been described in connection with the accompanying drawing. However, it should be understood that the figures are not drawn to scale. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

For purposes of this disclosure, certain aspects, advantages, and novel features are described herein. It is to be understood that not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

## Claims

1. A glaucoma treatment apparatus comprising:
an imaging device configured to capture pre-operative images of the anterior segment of the eye,
a femtosecond, FS, laser configured to actuate at a wavelength of about 1030 nm and apply laser pulses to the trabecular meshwork, TM, and the juxtacanicular tissue, JCT, of the eye, and
a hardware processor configured to:
receive a pre-operative intraocular pressure, IOP, level and a target, IOP, reduction; and
determine optimal location(s) and cross sectional area(s) of one or more aqueous humor, AH, drainage channels through the trabecular meshwork, TM, and
the juxtacanalicular tissue, JCT, of the eye based on at least:
an anatomy of the anterior segment of the eye obtained from pre-operative images of the anterior segment of the eye, said anatomy including the TM, the JCT, the Schlemm's Canal, SC, and collector channels, CCS,
a pre-operative intraocular pressure, IOP, level, and
a target IOP reduction,
wherein the hardware processor is configured to determine the optimal location(s) and cross-sectional area(s) of the one or more AH drainage channel(s) to be created by the FS laser in order to achieve the target IOP reduction by using a three-dimensional finite element model, FEM, computer model of AH outflow dynamics that uses the anatomy of the anterior segment of the treated eye, including the TM, SC and CCS obtained from pre-operative images, the measured IOP, and target IOP reduction as inputs,
wherein the FEM model assumes the following:
(i) all AH outflow occurs through the trabecular pathway;
(ii) the trabecular pathway in the eye is porous, so that outflow through the pathway follows the Brinkman equation and its resistance to outflow is characterized with permeability in the equation: $- v \left[η\left(v{u}_{_}+{\left(v{u}_{_}\right)}^{r}\right)\right] + vP + \frac{η}{K} {u}_{_} = 0 , v {u}_{_} = 0$ where v is a differential operator, u is a velocity vector (m/s), η is the dynamic viscosity in (Pa*second), P is pressure, (vu)r refers to the transposed velocity gradient vector and κ is permeability in m²;
(iii) the resistance of pathways through the TM, juxtacanalicular tissue, JCT, and inner wall endothelium of the SC are **characterized by** different permeability values;
(iv) the structures of the eye such as the cornea are rigid materials, and for the precise estimation of the AH outflow parameters deformation of the cornea and movement of the structural components such as lens and iris inside the eye are included in the FEM of the AH outflow; and
(v) impact of temperature variation on the AH outflow is not considered;
wherein the FEM is configured to determine the three-dimensional aqueous humor outflow dynamics of the pre-operative eye and the average permeability of the trabecular meshwork,
wherein the hardware processor is configured to use an inverse of the FEM to calculate the locations and necessary cross-sectional area(s) of the channel(s) to achieve the target IOP reduction; and
wherein the hardware processor is configured to actuate the laser in order to create the one or more drainage channel(s) through the TM and the JCT with the cross-sectional area(s) and location(s) determined in order to achieve the target intraocular pressure, IOP, reduction.

2. The glaucoma treatment apparatus according to claim 1, further comprising a suction ring configured to keep the apparatus steady on the eye during a treatment procedure.

3. The glaucoma treatment apparatus according to any one of the previous claims, wherein the apparatus is configured to automatically create drainage channels in between the anterior chamber, AC, and the Schlemm's canal, SC, based on output from the hardware processor.

4. The glaucoma treatment apparatus according to any one of the previous claims, wherein the treatment laser has a pulse duration ranging from 50 fs to 50 ns.

5. The glaucoma treatment apparatus according to any one of the previous claims, wherein the treatment laser has a repetition rate ranging up to 500 KHz.

6. The glaucoma treatment apparatus according to any one of the previous claims, wherein the imaging device is selected from the group consisting of a light microscope, an ultrasound device, a computed tomography, CT, device, a magnetic resonance imager, MRI, a system that utilizes molecular imaging, a system that utilizes nuclear medicine, a positron emission tomography, PET, system, a radiography system, and a fluoroscopy system.

7. A method for determining a location and a cross-sectional area of treatment in the trabecular meshwork of an eye, comprising:
acquiring a pre-operative image of the anterior segment of the eye, including the TM, JCT, SC and CCS, and
using an algorithm based on a customized anatomy of the anterior segment of the eye including the TM, JCT, SC and CCS obtained from the pre-operative images of the anterior segment of the eye, a pre-operative IOP level and a target IOP reduction as inputs to determine optimal location(s) and cross-sectional area(s) of one or more aqueous humor, AH, drainage channel(s) through the trabecular meshwork, TM, and the juxtacanalicular tissue, JCT, of the eye that will allow precise personalized control over intraocular pressure reduction,
wherein said determined optimal location(s) and cross-sectional area(s) are for targeting the TM and JCT with laser pulses of short durations with an FS laser configured to actuate at a wavelength of about 1030 nm to create the one or more drainage channel(s) through the TM and the JCT in between the anterior chamber, AC, and the Schlemm's canal, SC, said one or more drainage channel(s) being suitable for draining of aqueous humor from the anterior chamber to the SC to reduce intraocular pressure in the eye, wherein the one or more drainage channel(s) mediate precise and customized control over the magnitude and/or rate of drainage of the aqueous humor into the SC,
wherein the algorithm determines the size of the one or more drainage channel(s) necessary to achieve the target IOP reduction by using a three-dimensional finite element model, FEM, computer model of AH outflow dynamics that uses the anatomy of the anterior segment of the treated eye, including the TM, SC and CCS obtained from pre-operative images, the measured IOP and target IOP reduction as inputs,
wherein the FEM model assumes the following:
(i) all AH outflow occurs through the trabecular pathway;
(ii) the trabecular pathway in the eye is porous, so that outflow through the pathway follows the Brinkman equation and its resistance to outflow is characterized with permeability in the equation: $- v \left[η\left(v{u}_{_}+{\left(v{u}_{_}\right)}^{r}\right)\right] + vP + \frac{η}{K} {u}_{_} = 0 , v {u}_{_} = 0$ where v is a differential operator, u is a velocity vector (m/s), η is the dynamic viscosity in (Pa*second), P is pressure, (vu)r refers to the transposed velocity gradient vector and κ is permeability in m²;
(iii) the resistance of pathways through the TM, juxtacanalicular tissue, JCT, and inner wall endothelium of the SC are **characterized by** different permeability values;
(iv) the structures of the eye such as the cornea are rigid materials, and for the precise estimation of the AH outflow parameters deformation of the cornea and movement of the structural components such as lens and iris inside the eye are included in the FEM of the AH outflow; and
(v) impact of temperature variation on the AH outflow is not considered; and
inputting the target IOP; and
solving an inverse of the FEM to determine the one or more location(s) and a cross-sectional area(s) of one or more drainage channel(s) through the TM and the JCT with the cross-sectional area(s) and location(s) in order to achieve the target intraocular pressure IOP, reduction, wherein three-dimensional aqueous humor outflow dynamics of the pre-operative eye based on a customized anatomy of the anterior segment including the TM and SC obtained from pre-operative images are determined by the FEM and wherein the average permeability of the trabecular meshwork is determined by the FEM.

8. The method according to claim 7, wherein:
the laser pulses of short durations used for the treatment range from 50 fs to 50 ns, and/or
a repetition rate of the laser pulses of short durations used for the treatment ranges up to 500 KHz.

9. The method according to any one of claims 7-8, wherein in the algorithm, the anterior chamber is modeled as a hemisphere.

10. The method according to claim 9, wherein the hemisphere is modeled with a diameter of 12 mm along the edge of the iris.

11. The method according to any one of claims 7-10, wherein in the algorithm, the height of the trabecular meshwork, TM, is modeled to range from 0.38 to 0.86 mm.

12. The method according to claim 11, wherein in the algorithm, the height of the trabecular meshwork, TM, is modeled to have an average height of 0.6 mm.

13. The method according to any one of claims 7-12, wherein in the algorithm, the final destination of aqueous humor, AH, flow is modeled to have a constant pressure boundary condition of 9.6 mmHg, corresponding to the back-pressure in the episcleral venous system.

14. Use of the glaucoma treatment apparatus according to any one of claims 1-6 to calculate the dimensions and the positioning of the one or more drainage channel(s) to be created by targeting the trabecular meshwork, TM, and the juxtacanalicular tissue JCT, with laser pulses of short durations at a wavelength of about 1030 nm to create the one or more drainage channel(s) through the TM and the JCT, in between the anterior chamber, AC, and the Schlemm's canal, SC, wherein the dimensions and positioning of the one or more drainage channel(s) are calculated using an algorithm based on a customized anatomy of the anterior segment of the eye including the TM, JCT, SC and CCS obtained from the pre-operative images of the anterior segment of the eye, a preoperative IOP level and a target IOP reduction as inputs to determine a location and a cross-sectional area of the trabecular meshwork and JCT of the eye that will allow precise personalized control over intraocular pressure reduction.

## Patentansprüche

1. Glaukombehandlungseinrichtung, umfassend:
eine Bildgebungsvorrichtung, die dazu ausgebildet ist, präoperative Bilder des vorderen Augenabschnitts aufzunehmen, einen Femtosekundenlaser, FS-Laser, der dazu ausgebildet ist, bei einer Wellenlänge von etwa 1030 nm betätigt zu werden und Laserpulse auf das Trabekelwerk, TM, und das juxtakanalikuläre Gewebe, JCT, des Auges aufzubringen, und
einen Hardwareprozessor, der dazu ausgebildet ist:
einen präoperativen Augeninnendruckwert, IOP-Wert, und eine angestrebte, IOP, Senkung zu empfangen; und
optimale Position(en) und Querschnittsfläche(n) eines oder mehrerer Kammerwasserabflusskanäle, AH-Abflusskanäle, durch das Trabekelwerk, TM, und
das juxtakanalikuläre Gewebe, JCT, des Auges zu bestimmen, basierend auf mindestens:
einer Anatomie des vorderen Augenabschnitts, die aus präoperativen Bildern des vorderen Augenabschnitts gewonnen wird, wobei die genannte Anatomie das TM, das JCT, den Schlemm-Kanal, SC, und Sammelkanäle, CCS, umfasst,
einem präoperativen Augeninnendruckwert, IOP-Wert, und
einer angestrebten IOP-Senkung,
wobei der Hardwareprozessor dazu ausgebildet ist, die optimale(n) Position(en) und Querschnittsfläche(n) des einen oder der mehreren AH-Abflusskanäle, die durch den FS-Laser zu erzeugen sind, zu bestimmen, um die angestrebte IOP-Senkung zu erreichen, indem ein dreidimensionales Finite-Elemente-Modell-Computermodell, FEM-Computermodell, der AH-Abflussdynamik verwendet wird, das die Anatomie des vorderen Augenabschnitts des behandelten Auges, einschließlich des TM, des SC und der CCS, die aus präoperativen Bildern gewonnen werden, den gemessenen IOP und die angestrebte IOP-Senkung als Eingaben verwendet,
wobei das FEM-Modell Folgendes annimmt:
(i) der gesamte AH-Abfluss erfolgt über den trabekulären Weg;
(ii) der trabekuläre Weg im Auge ist porös, sodass der Abfluss durch den Weg der Brinkman-Gleichung folgt und sein Abflusswiderstand durch die Permeabilität in der Gleichung charakterisiert wird: $- v \left[η\left(v{u}_{_}+{\left(v{u}_{_}\right)}^{r}\right)\right] + vP + \frac{η}{K} {u}_{_} = 0 , v {u}_{_} = 0$ wobei v ein Differentialoperator ist, u ein Geschwindigkeitsvektor (m/s) ist, η die dynamische Viskosität in (Pa* Sekunde) ist, P der Druck ist, (vu)r sich auf den transponierten Geschwindigkeitsgradientenvektor bezieht und κ die Permeabilität in m² ist;
(iii) der Widerstand der Wege durch das TM, das juxtakanalikuläre Gewebe, JCT, und das Endothel der Innenwand des SC ist durch unterschiedliche Permeabilitätswerte charakterisiert;
(iv) die Strukturen des Auges wie etwa die Hornhaut sind starre Materialien, und zur genauen Abschätzung der AH-Abflussparameter sind eine Verformung der Hornhaut und eine Bewegung der Strukturkomponenten wie etwa Linse und Iris im Inneren des Auges in das FEM des AH-Abflusses einbezogen; und
(v) ein Einfluss von Temperaturschwankungen auf den AH-Abfluss wird nicht berücksichtigt;
wobei das FEM dazu ausgebildet ist, die dreidimensionale Kammerwasserabflussdynamik des präoperativen Auges und die durchschnittliche Permeabilität des Trabekelwerks zu bestimmen,
wobei der Hardwareprozessor dazu ausgebildet ist, eine Inverse des FEM zu verwenden, um die Positionen und notwendigen Querschnittsfläche(n) des Kanals/der Kanäle zu berechnen, um die angestrebte IOP-Senkung zu erreichen; und
wobei der Hardwareprozessor dazu ausgebildet ist, den Laser zu betätigen, um den einen oder die mehreren Abflusskanäle durch das TM und das JCT mit der/den bestimmten Querschnittsfläche(n) und Position(en) zu erzeugen, um die angestrebte Senkung des Augeninnendrucks, IOP, zu erreichen.

2. Glaukombehandlungseinrichtung nach Anspruch 1, ferner umfassend einen Saugring, der dazu ausgebildet ist, die Einrichtung während eines Behandlungsvorgangs stabil auf dem Auge zu halten.

3. Glaukombehandlungseinrichtung nach einem der vorhergehenden Ansprüche, wobei die Einrichtung dazu ausgebildet ist, basierend auf einer Ausgabe des Hardwareprozessors automatisch Abflusskanäle zwischen der Vorderkammer, AC, und dem Schlemm-Kanal, SC, zu erzeugen.

4. Glaukombehandlungseinrichtung nach einem der vorhergehenden Ansprüche, wobei der Behandlungslaser eine Pulsdauer im Bereich von 50 fs bis 50 ns aufweist.

5. Glaukombehandlungseinrichtung nach einem der vorhergehenden Ansprüche, wobei der Behandlungslaser eine Wiederholrate im Bereich bis zu 500 kHz aufweist.

6. Glaukombehandlungseinrichtung nach einem der vorhergehenden Ansprüche, wobei die Bildgebungsvorrichtung ausgewählt ist aus der Gruppe bestehend aus einem Lichtmikroskop, einer Ultraschallvorrichtung, einer Computertomographievorrichtung, CT-Vorrichtung, einem Magnetresonanztomographen, MRT, einem System, das molekulare Bildgebung nutzt, einem System, das Nuklearmedizin nutzt, einem Positronenemissionstomographie-System, PET-System, einem Radiographiesystem und einem Durchleuchtungssystem.

7. Verfahren zum Bestimmen einer Position und einer Querschnittsfläche einer Behandlung im Trabekelwerk eines Auges, umfassend:
Erfassen eines präoperativen Bildes des vorderen Augenabschnitts, einschließlich des TM, des JCT, des SC und der CCS, und
Verwenden eines Algorithmus, der auf einer angepassten Anatomie des vorderen Augenabschnitts, einschließlich des TM, des JCT, des SC und der CCS, die aus den präoperativen Bildern des vorderen Augenabschnitts gewonnen werden, einem präoperativen IOP-Wert und einer angestrebten IOP-Senkung als Eingaben basiert, um optimale Position(en) und Querschnittsfläche(n) eines oder mehrerer Kammerwasserabflusskanäle, AH-Abflusskanäle, durch das Trabekelwerk, TM, und das juxtakanalikuläre Gewebe, JCT, des Auges zu bestimmen, die eine präzise personalisierte Steuerung der Augeninnendrucksenkung ermöglichen,
wobei die genannten bestimmten optimalen Position(en) und Querschnittsfläche(n) dazu dienen, das TM und das JCT mit Laserpulsen kurzer Dauer mit einem FS-Laser, der dazu ausgebildet ist, bei einer Wellenlänge von etwa 1030 nm betätigt zu werden, gezielt zu beaufschlagen, um den einen oder die mehreren Abflusskanäle durch das TM und das JCT zwischen der Vorderkammer, AC, und dem Schlemm-Kanal, SC, zu erzeugen, wobei der genannte eine oder die genannten mehreren Abflusskanäle dazu geeignet sind, Kammerwasser aus der Vorderkammer in den SC abzuleiten, um den Augeninnendruck im Auge zu senken, wobei der eine oder die mehreren Abflusskanäle eine präzise und angepasste Steuerung des Ausmaßes und/oder der Rate des Abflusses des Kammerwassers in den SC vermitteln,
wobei der Algorithmus die Größe des einen oder der mehreren Abflusskanäle bestimmt, die notwendig ist, um die angestrebte IOP-Senkung zu erreichen, indem ein dreidimensionales Finite-Elemente-Modell-Computermodell, FEM-Computermodell, der AH-Abflussdynamik verwendet wird, das die Anatomie des vorderen Augenabschnitts des behandelten Auges, einschließlich des TM, des SC und der CCS, die aus präoperativen Bildern gewonnen werden, den gemessenen IOP und die angestrebte IOP-Senkung als Eingaben verwendet,
wobei das FEM-Modell Folgendes annimmt:
(i) der gesamte AH-Abfluss erfolgt über den trabekulären Weg;
(ii) der trabekuläre Weg im Auge ist porös, sodass der Abfluss durch den Weg der Brinkman-Gleichung folgt und sein Abflusswiderstand durch die Permeabilität in der Gleichung charakterisiert wird: $- v \left[η\left(v{u}_{_}+{\left(v{u}_{_}\right)}^{r}\right)\right] + vP + \frac{η}{K} {u}_{_} = 0 , v {u}_{_} = 0$ wobei v ein Differentialoperator ist, u ein Geschwindigkeitsvektor (m/s) ist, η die dynamische Viskosität in (Pa*Sekunde) ist, P der Druck ist, (vu)r sich auf den transponierten Geschwindigkeitsgradientenvektor bezieht und κ die Permeabilität in m² ist;
(iii) der Widerstand der Wege durch das TM, das juxtakanalikuläre Gewebe, JCT, und das Endothel der Innenwand des SC ist durch unterschiedliche Permeabilitätswerte charakterisiert;
(iv) die Strukturen des Auges wie etwa die Hornhaut sind starre Materialien, und zur genauen Abschätzung der AH-Abflussparameter sind eine Verformung der Hornhaut und eine Bewegung der Strukturkomponenten wie etwa Linse und Iris im Inneren des Auges in das FEM des AH-Abflusses einbezogen; und
(v) ein Einfluss von Temperaturschwankungen auf den AH-Abfluss wird nicht berücksichtigt; und
Eingeben des angestrebten IOP; und
Lösen einer Inversen des FEM, um die eine oder die mehreren Position(en) und eine Querschnittsfläche(n) eines oder mehrerer Abflusskanäle durch das TM und das JCT mit der/den Querschnittsfläche(n) und Position(en) zu bestimmen, um die angestrebte Senkung des Augeninnendrucks, IOP, zu erreichen, wobei eine dreidimensionale Kammerwasserabflussdynamik des präoperativen Auges basierend auf einer angepassten Anatomie des vorderen Augenabschnitts, einschließlich des TM und des SC, die aus präoperativen Bildern gewonnen werden, durch das FEM bestimmt wird und wobei die durchschnittliche Permeabilität des Trabekelwerks durch das FEM bestimmt wird.

8. Verfahren nach Anspruch 7, wobei:
die für die Behandlung verwendeten Laserpulse kurzer Dauer im Bereich von 50 fs bis 50 ns liegen und/oder
eine Wiederholrate der für die Behandlung verwendeten Laserpulse kurzer Dauer im Bereich bis zu 500 kHz liegt.

9. Verfahren nach einem der Ansprüche 7-8, wobei im Algorithmus die Vorderkammer als Halbkugel modelliert wird.

10. Verfahren nach Anspruch 9, wobei die Halbkugel mit einem Durchmesser von 12 mm entlang des Irisrands modelliert wird.

11. Verfahren nach einem der Ansprüche 7-10, wobei im Algorithmus die Höhe des Trabekelwerks, TM, so modelliert wird, dass sie im Bereich von 0,38 bis 0,86 mm liegt.

12. Verfahren nach Anspruch 11, wobei im Algorithmus die Höhe des Trabekelwerks, TM, so modelliert wird, dass sie eine durchschnittliche Höhe von 0,6 mm aufweist.

13. Verfahren nach einem der Ansprüche 7-12, wobei im Algorithmus der endgültige Bestimmungsort des Kammerwasserflusses, AH-Flusses, so modelliert wird, dass er eine konstante Druckrandbedingung von 9,6 mmHg aufweist, die dem Gegendruck im episkleralen Venensystem entspricht.

14. Verwendung der Glaukombehandlungseinrichtung nach einem der Ansprüche 1-6 zum Berechnen der Abmessungen und der Positionierung des einen oder der mehreren zu erzeugenden Abflusskanäle, indem das Trabekelwerk, TM, und das juxtakanalikuläre Gewebe, JCT, mit Laserpulsen kurzer Dauer bei einer Wellenlänge von etwa 1030 nm gezielt beaufschlagt werden, um den einen oder die mehreren Abflusskanäle durch das TM und das JCT zwischen der Vorderkammer, AC, und dem Schlemm-Kanal, SC, zu erzeugen, wobei die Abmessungen und die Positionierung des einen oder der mehreren Abflusskanäle mithilfe eines Algorithmus berechnet werden, der auf einer angepassten Anatomie des vorderen Augenabschnitts, einschließlich des TM, des JCT, des SC und der CCS, die aus den präoperativen Bildern des vorderen Augenabschnitts gewonnen werden, einem präoperativen IOP-Wert und einer angestrebten IOP-Senkung als Eingaben basiert, um eine Position und eine Querschnittsfläche des Trabekelwerks und des JCT des Auges zu bestimmen, die eine präzise personalisierte Steuerung der Augeninnendrucksenkung ermöglichen.

## Revendications

1. Appareil de traitement du glaucome comprenant :
un dispositif d'imagerie configuré pour capturer des images préopératoires du segment antérieur de l'œil,
un laser femtoseconde, FS, configuré pour fonctionner à une longueur d'onde d'environ 1 030 nm et appliquer des impulsions laser au trabéculum, TM, et au tissu juxtacaniculaire, JCT, de l'œil, et
un processeur matériel configuré pour :
recevoir un niveau de pression intraoculaire préopératoire, PIO, et une réduction cible de la PIO ; et
déterminer un ou des emplacements et une ou des surfaces de section transversale optimaux d'un ou de plusieurs canaux de drainage de l'humeur aqueuse, HA, à travers le trabéculum, TM, et
le tissu juxtacanaliculaire, JCT, de l'œil, sur la base au moins des éléments suivants :
une anatomie du segment antérieur de l'œil obtenue à partir d'images préopératoires du segment antérieur de l'œil, ladite anatomie comportant le TM, le JCT, le canal de Schlemm, CS, et les canaux collecteurs, CC,
un niveau de pression intraoculaire préopératoire, PIO, et
une réduction cible de la PIO,
dans lequel le processeur matériel est configuré pour déterminer le ou les emplacements et la ou les surfaces de section transversale optimaux des un ou plusieurs canaux de drainage de l'HA à créer par le laser FS afin d'atteindre la réduction cible de la PIO à l'aide d'un modèle informatique tridimensionnel par éléments finis, MEF, de la dynamique d'écoulement de l'HA qui utilise l'anatomie du segment antérieur de l'œil traité, comportant le TM, le CS et les CC, obtenue à partir d'images préopératoires, la PIO mesurée et la réduction cible de la PIO comme entrées,
dans lequel le modèle MEF suppose ce qui suit :
(i) tout l'écoulement de l'HA se produit par la voie trabéculaire ;
(ii) la voie trabéculaire de l'œil est poreuse, de sorte que l'écoulement à travers la voie suit l'équation de Brinkman et sa résistance à l'écoulement est **caractérisée par** la perméabilité dans l'équation : $- v \left[η\left(v{u}_{_}+{\left(v{u}_{_}\right)}^{r}\right)\right] + vP + \frac{η}{K} {u}_{_} = 0 , v {u}_{_} = 0$ où v est un opérateur différentiel, u est un vecteur de vitesse (m/s), η est la viscosité dynamique en (Pa* seconde), P est la pression, (vu)r fait référence au vecteur gradient de vitesse transposé et κ est la perméabilité en m² ;
(iii) la résistance des voies à travers le TM, le tissu juxtacanaliculaire, JCT, et l'endothélium de la paroi interne du CS est **caractérisée par** des valeurs de perméabilité différentes ;
(iv) les structures de l'œil telles que la cornée sont des matériaux rigides, et pour l'estimation précise des paramètres d'écoulement de l'HA, la déformation de la cornée et le mouvement des composants structurels tels que le cristallin et l'iris à l'intérieur de l'œil sont inclus dans le MEF de l'écoulement de l'HA ; et
(v) l'impact de la variation de température sur l'écoulement de l'HA n'est pas pris en compte ;
dans lequel le MEF est configuré pour déterminer la dynamique tridimensionnelle de l'écoulement de l'humeur aqueuse de l'œil préopératoire et la perméabilité moyenne du trabéculum,
dans lequel le processeur matériel est configuré pour utiliser l'inverse du MEF afin de calculer les emplacements et la ou les surfaces de section transversale nécessaires du ou des canaux pour atteindre la réduction cible de la PIO ; et
dans lequel le processeur matériel est configuré pour actionner le laser afin de créer les un ou plusieurs canaux de drainage à travers le TM et le JCT, la ou les surfaces de section transversale et le ou les emplacements étant déterminés afin d'atteindre la réduction cible de la pression intraoculaire, PIO.

2. Appareil de traitement du glaucome selon la revendication 1, comprenant en outre un anneau d'aspiration configuré pour maintenir l'appareil stable sur l'œil pendant une procédure de traitement.

3. Appareil de traitement du glaucome selon l'une quelconque des revendications précédentes, dans lequel l'appareil est configuré pour créer automatiquement des canaux de drainage entre la chambre antérieure, CA, et le canal de Schlemm, CS, sur la base de la sortie du processeur matériel.

4. Appareil de traitement du glaucome selon l'une quelconque des revendications précédentes, dans lequel le laser de traitement a une durée d'impulsion allant de 50 fs à 50 ns.

5. Appareil de traitement du glaucome selon l'une quelconque des revendications précédentes, dans lequel le laser de traitement a une fréquence de répétition allant jusqu'à 500 kHz.

6. Appareil de traitement du glaucome selon l'une quelconque des revendications précédentes, dans lequel le dispositif d'imagerie est choisi parmi le groupe constitué d'un microscope optique, d'un appareil à ultrasons, d'un dispositif de tomodensitométrie, TDM, d'un appareil d'imagerie par résonance magnétique, IRM, d'un système qui utilise l'imagerie moléculaire, d'un système qui utilise la médecine nucléaire, d'un système de tomographie par émission de positons, TEP, d'un système de radiographie et d'un système de fluoroscopie.

7. Procédé de détermination d'un emplacement et d'une surface de section transversale de traitement dans le trabéculum d'un œil, comprenant :
l'acquisition d'une image préopératoire du segment antérieur de l'œil, comportant le TM, le JCT, le CS et les CC, et
l'utilisation d'un algorithme sur la base d'une anatomie personnalisée du segment antérieur de l'œil comportant le TM, le JCT, le CS et les CC, obtenue à partir des images préopératoires du segment antérieur de l'œil, un niveau de PIO préopératoire et une réduction cible de la PIO comme entrées pour déterminer un ou des emplacements et une ou des surfaces de section transversale optimaux d'un ou de plusieurs canaux de drainage de l'humeur aqueuse, HA, à travers le trabéculum, TM, et le tissu juxtacanaliculaire, JCT, de l'œil, ce qui permettra un contrôle personnalisé précis de la réduction de la pression intraoculaire,
dans lequel lesdits emplacements et surfaces de section transversale optimaux déterminés servent à cibler le TM et le JCT avec des impulsions laser de courte durée à l'aide d'un laser FS configuré pour fonctionner à une longueur d'onde d'environ 1 030 nm afin de créer les un ou plusieurs canaux de drainage à travers le TM et le JCT entre la chambre antérieure, CA, et le canal de Schlemm, CS, lesdits un ou plusieurs canaux de drainage étant adaptés au drainage de l'humeur aqueuse de la chambre antérieure vers le CS afin de réduire la pression intraoculaire dans l'œil, dans lequel les un ou plusieurs canaux de drainage permettent un contrôle précis et personnalisé de l'amplitude et/ou du débit de drainage de l'humeur aqueuse dans le CS,
dans lequel l'algorithme détermine la taille des un ou plusieurs canaux de drainage nécessaires pour atteindre la réduction cible de la PIO à l'aide d'un modèle informatique tridimensionnel par éléments finis, MEF, de la dynamique d'écoulement de l'HA qui utilise l'anatomie du segment antérieur de l'œil traité, comportant le TM, le CS et les CC, obtenue à partir d'images préopératoires, la PIO mesurée et la réduction cible de la PIO comme entrées,
dans lequel le modèle MEF suppose ce qui suit :
(i) tout l'écoulement de l'HA se produit par la voie trabéculaire ;
(ii) la voie trabéculaire de l'œil est poreuse, de sorte que l'écoulement à travers la voie suit l'équation de Brinkman et sa résistance à l'écoulement est **caractérisée par** la perméabilité dans l'équation : $- v \left[η\left(v{u}_{_}+{\left(v{u}_{_}\right)}^{r}\right)\right] + vP + \frac{η}{K} {u}_{_} = 0 , v {u}_{_} = 0$ où v est un opérateur différentiel, u est un vecteur de vitesse (m/s), η est la viscosité dynamique en (Pa*seconde), P est la pression, (vu)r fait référence au vecteur gradient de vitesse transposé et κ est la perméabilité en m² ;
(iii) la résistance des voies à travers le TM, le tissu juxtacanaliculaire, JCT, et l'endothélium de la paroi interne du CS est **caractérisée par** des valeurs de perméabilité différentes ;
(iv) les structures de l'œil telles que la cornée sont des matériaux rigides, et pour l'estimation précise des paramètres d'écoulement de l'HA, la déformation de la cornée et le mouvement des composants structurels tels que le cristallin et l'iris à l'intérieur de l'œil sont inclus dans le MEF de l'écoulement de l'HA ; et
(v) l'impact de la variation de température sur l'écoulement de l'HA n'est pas pris en compte ; et
la saisie de la PIO cible ; et
la résolution de l'inverse du MEF pour déterminer les un ou plusieurs emplacements et une ou plusieurs surfaces de section transversale d'un ou de plusieurs canaux de drainage à travers le TM et le JCT avec la ou les surfaces de section transversale et le ou les emplacements afin d'atteindre la réduction cible de la pression intraoculaire, PIO, dans lequel la dynamique tridimensionnelle de l'écoulement de l'humeur aqueuse de l'œil préopératoire, sur la base d'une anatomie personnalisée du segment antérieur comportant le TM et le CS, obtenue à partir d'images préopératoires, est déterminée par le MEF et dans lequel la perméabilité moyenne du trabéculum est déterminée par le MEF.

8. Procédé selon la revendication 7, dans lequel :
les impulsions laser de courte durée utilisées pour le traitement varient de 50 fs à 50 ns, et/ou
la fréquence de répétition des impulsions laser de courte durée utilisées pour le traitement peut aller jusqu'à 500 kHz.

9. Procédé selon l'une quelconque des revendications 7 à 8, dans lequel, dans l'algorithme, la chambre antérieure est modélisée comme un hémisphère.

10. Procédé selon la revendication 9, dans lequel l'hémisphère est modélisé avec un diamètre de 12 mm le long du bord de l'iris.

11. Procédé selon l'une quelconque des revendications 7 à 10, dans lequel, dans l'algorithme, la hauteur du trabéculum, TM, est modélisée pour varier de 0,38 à 0,86 mm.

12. Procédé selon la revendication 11, dans lequel, dans l'algorithme, la hauteur du trabéculum, TM, est modélisée comme ayant une hauteur moyenne de 0,6 mm.

13. Procédé selon l'une quelconque des revendications à 7 à 12, dans lequel, dans l'algorithme, la destination finale du flux d'humeur aqueuse, HA, est modélisée comme ayant une condition limite de pression constante de 9,6 mmHg, correspondant à la contre-pression dans le système veineux épiscléral.

14. Utilisation de l'appareil de traitement du glaucome selon l'une quelconque des revendications 1 à 6 pour calculer les dimensions et le positionnement des un ou plusieurs canaux de drainage à créer en ciblant le trabéculum, TM, et le tissu juxtacanaliculaire, JCT, avec des impulsions laser de courte durée à une longueur d'onde d'environ 1 030 nm afin de créer les un ou plusieurs canaux de drainage à travers le TM et le JCT, entre la chambre antérieure, CA, et le canal de Schlemm, CS, dans laquelle les dimensions et le positionnement des un ou plusieurs canaux de drainage sont calculés à l'aide d'un algorithme sur la base d'une anatomie personnalisée du segment antérieur de l'œil comportant le TM, le JCT, le CS et les CC, obtenue à partir des images préopératoires du segment antérieur de l'œil, un niveau de PIO préopératoire et une réduction cible de la PIO comme entrées pour déterminer un emplacement et une surface de section transversale du trabéculum et du JCT de l'œil permettant un contrôle personnalisé précis de la réduction de la pression intraoculaire.
